# FASCICULE DE BREVET EUROPEEN

(11) **EP 1 692 162 B1**
(45) Date de publication et mention de la délivrance du brevet: **13.01.2010**
(21) Numéro de dépôt: 04805807.7
(22) Date de dépôt: 04.11.2004
(51) Int. Cl.: C07K 14/21, C12N 15/78, C12N 15/62, C07K 19/00, C12N 1/21

(54) **OUTIL DE TRANSFERT ET DE PRODUCTION DE PROTEINES METTANT EN OEUVRE LE SYSTEME DE SECRETION DE TYPE III DE PSEUDOMONAS**
TOOL FÜR DEN TRANSFER UND DIE HERSTELUNG VON PROTEINEN MIT DEM PSEUDOMONAS-TYP-III-SEKRETIONSSYSTEM
TOOL FOR THE TRANSFER AND PRODUCTION OF PROTEINS USING THE PSEUDOMONAS TYPE III SECRETION SYSTEM

(30) Priorité: 13.11.2003 FR 0313286
(43) Date de publication de la demande: 23.08.2006
(73) Titulaire: Université Joseph Fourier, 38041 Grenoble Cédex 9 (FR)
(72) Inventeur: POLACK, Benoît, F-38950 Saint Martin Le Vinoux (FR); TOUSSAINT, Bertrand, F-38120 Saint Egrève (FR); QUENEE, Lauriane, F-38700 La Tronche (FR)
(74) Mandataire: Vuillermoz, Bruno
(86) Numéro de dépôt international: PCT/FR2004/050564
(87) Numéro de publication internationale: WO 2005/049644

(56) Documents cités:
- BENOÎT POLACK ET AL.: "Protein delivery by Pseudomonas type III secretion system: Ex vivo complementation of P67phox-deficient chronic granulomatous disease" BIOCHEMICAL AND BIOPHYSICAL RESEARCH COMMUNICATIONS, vol. 275, 2000, pages 854-858, XP002284083 cité dans la demande
- KRISTIN J. PEDERSON ET AL.: "Intracellular localization and processing of Pseudomonas aeruginosa ExoS in eukaryotic cells" MOLECULAR MICROBIOLOGY, vol. 37, no. 2, 2000, pages 287-299, XP002284084
- KRISTIN J. PEDERSON ET AL.: "Intracellular localization modulates targeting of ExoS, a type III cytotoxin, to eukaryotic signalling proteins" MOLECULAR MICROBIOLOGY, vol. 46, no. 5, 2002, pages 1381-1390, XP002284085
- R. KRALL ET AL.: "Intracellular targeting of two type-III secreted cytokines" ABSTRACTS OF THE GENERAL MEETING OF THE AMERICAN SOCIETY FOR MICROBIOLOGY, vol. 103, mai 2003 (2003-05), pages 42-43, XP008031646

## Description

L'invention concerne un nouvel outil de transfert dans des cellules eucaryotes ou de production dans le surnageant d'une culture bactérienne, de protéines chimères à partir du système de sécrétion du type III présent chez la bactérie *Pseudomonas,* en particulier *Pseudomonas aeruginosa.* Plus précisément, l'invention se rapporte à de nouveaux vecteurs d'expression de protéines chimères ou, plus généralement, de séquences d'acides aminés, dans *P. aeruginosa.* Elle concerne également des souches de *P. aeruginosa* transformées avec lesdits vecteurs d'expression en vue de la sécrétion ou de l'injection, par le SSTT de *P. aeruginosa,* de protéines chimères dans le surnageant de culture ou dans des cellules eucaryotes respectivement. Elle a donc également pour objet une méthode d'injection (dénommée également translocation) *in vitro* ou *ex vivo* dans des cellules eucaryotes, de protéines chimères, de même qu'une méthode de production de protéines chimères dans le surnageant de culture, méthodes pouvant être utilisées à des fins thérapeutiques.

Les bactéries possèdent naturellement des systèmes de sécrétion de protéines qui leur permettent d'envoyer à distance des enzymes ou des toxines. En plus de ces systèmes, certaines d'entre-elles possèdent un système de sécrétion, dit "de type III" (dénommé SSTT) qui leur permet d'injecter directement des protéines toxiques, depuis leur cytosol jusque dans le cytosol d'une cellule cible. Cet appareil de sécrétion est un système complexe, constitué d'un certain nombre d'éléments formant un canal traversant la membrane interne, l'espace périplasmique et la membrane externe de la bactérie, ce canal permettant le passage de toxines spécifiques. Dans la plupart des espèces bactériennes, ce système de sécrétion peut être activé soit par contact avec la cellule eucaryote cible, ce qui entraîne l'injection des toxines dans le cytosol de la cellule cible, soit par la chélation du Ca²⁺ dans le milieu extérieur, ce qui entraîne la sécrétion des toxines spécifiques dans le milieu de culture.

Il est donc paru intéressant d'étudier si les systèmes de sécrétion du type III (SSTT) pouvaient être utilisés pour l'injection de protéines d'intérêt dans des cellules eucaryotes en fusionnant la séquence nucléotidique codant la toxine secrétée par le SSTT avec une seconde séquence nucléotidique codant une protéine d'intérêt.

Un SSTT a été décrit chez les bactéries du genre *Yersinia* par Cornelis et Al. **[1]** (1997). Ce document indique en effet que le système de sécrétion de type III de *Yersinia entérocolitica,* dénommé Ysc, est capable d'assurer la sécrétion puis la translocation dans une cellule hôte de protéines effectrices, notamment YopE et YopH. SORY et Al. **[2]** ont étudié la structure moléculaire des protéines Yop et mis en évidence la présence, sur YopE et YopH de trois domaines, respectivement un domaine N-terminal nécessaire à la sécrétion, un domaine nécessaire à la translocation et une partie C-terminale, responsable de l'activité toxique de ladite protéine. Ces propriétés ont été mises en évidence par la construction de protéines hybrides obtenues par fusion de la partie amino-terminale de YopE ou YopH avec une enzyme rapportrice, telle que par exemple l'adénylcylase activée par la calmoduline, l'activité enzymatique étant ensuite mesurée.

Le document US-A-5 965 381 mentionne par ailleurs l'intérêt d'identifier précisément la taille minimale de la séquence nucléotidique codant la protéine effectrice nécessaire à la sécrétion puis au transfert d'une protéine cible dans une cellule eucaryote. Pour ce faire, la méthode décrite consiste, de la même manière que précédemment évoquée, à fusionner des séquences comprenant un nombre décroissant de nucléotides avec une séquence codant une enzyme rapportrice activée par la calmoduline, l'apparition d'une activité cyclasique dans le cytosol de la cellule transloquée témoignant de l'existence de la translocation. Même s'il ressort de la description que les constructions ont été réalisées à partir des 50, 71, 100 et 130 premiers acides aminés de YopE, seul le fragment de 130 acides aminés est illustré, ce qui suppose que les autres séquences d'acides aminés identifiées sont trop courtes pour permettre la sécrétion puis la translocation de la protéine d'intérêt. En conséquence, le nombre élevé d'acides aminés nécessaire à la sécrétion (130 acides aminés) limite, sinon interdit la possible utilisation de cette séquence pour le transfert de protéines d'intérêt de taille importante.

Le document US-B-6 306 387 rapporte la présence d'un système de sécrétion du type III dans les bactéries du genre *Salmonella.* Ce document démontre en particulier la possibilité de transloquer, dans des cellules hôtes, une protéine chimérique dénommée SptP-NPc obtenue par fusion des 173 acides aminés N-terminaux constitutifs de la protéine toxique SptP sécrétée naturellement par le système de type III de *Salmonella* avec une séquence partielle codant une nucléoprotéique du virus *Influenza,* la détection étant réalisée au moyen d'anticorps capables de détecter la protéine chimérique dans la cellule infectée. Là encore, la séquence d'acides aminés de la protéine toxique injectée est relativement longue puisqu'elle ne compte pas moins d'au minimum 173 acides aminés interdisant donc le transfert dans une cellule hôte, de grosses protéines fusionnées. En effet, les protéines injectées n'étant pas ultérieurement clivées, il est nécessaire de disposer d'une protéine toxique de taille la plus faible possible.

L'existence d'un système de sécrétion du type III a également été mis en évidence dans un troisième genre de bactéries qui est le genre *Pseudomonas,* notamment les bactéries du type *Pseudonwnas aeruginosa. P. aeruginosa* est un bacille Gram négatif, pathogène opportuniste, responsable d'infections nosocomiales graves dans les hôpitaux. Il constitue également l'une des principales causes de morbidité et de mortalité chez les personnes atteintes de mucoviscidose. Le système de sécrétion de type III de *P*. *aeruginosa* constitue un moyen de défense très efficace, en particulier contre les phagocytes, notamment les granulocytes neutrophiles, première ligne de défense de l'immunité cellulaire. Ce système est également utilisé par la bactérie pour induire la prolifération et l'apoptose des lymphocytes T. On sait que *P. aeruginosa* sécrète quatre toxines grâce au système de sécrétion du type III, dénommées respectivement ExoS, ExoT, ExoY et ExoU.

ExoS est une protéine dont la séquence constituée de 454 AA est connue et publiée dans la GenBank. En amont du gène codant ExoS, il existe une séquence nucléotidique pouvant coder une protéine chaperonne supposée spécifique de ExoS, dénommée « Orf 1 ». ExoS possède une activité d'ADP-ribosylation exercée sur les petites protéines G de la famille Ras, ainsi qu'une capacité à activer les Rho-GTPases. ExoT possède quant à lui, également une activité ADP-ribosylante, toutefois 100 fois moindre que ExoS. En outre, on sait que ExoS et ExoT ont des séquences nucléotidiques identiques à 75 % (selon le programme LFASTA, W.R. Pearson & D.J. Lipman PNAS (1998) 85 : 2444-2448). Le mécanisme d'action d'ExoU n'est pas connu, mais il s'agit d'une cytotoxine puissante sur les épithéliums et les macrophages.

Le document Polack **[3]** fait référence à une souche naturelle isolée de *P. aeruginosa* dont les caractéristiques ne sont pas mentionnées (souche CHA) transformée avec un plasmide pBP31 contenant essentiellement deux séquences nucléotidiques fusionnées correspondant respectivement à la séquence nucléotidique codant les 129 acides aminés N-terminaux de ExoS et la séquence codant la protéine p67^{phox} impliquée dans la reconstitution d'un complexe enzymatique NADPH oxydase. Polack démontre la capacité de cette souche transformée à non seulement sécréter la protéine p67 mais également à reconstituer l'activité NADPH oxydase après injection de la protéine fusionnée dans le cytosol des lymphocytes B-EBV, témoignant ainsi de l'activité intracellulaire des protéines injectées. La séquence de 129 AA de Exo S utilisée par Polack est donc la séquence la plus courte jamais décrite pour l'injection de protéines cibles.

Par ailleurs, KRISTEN J. PEDERSON et al. (Mol. Microbiol. Vol 46, n° 5, 2002, pp 1381-1390) ont décrit un vecteur portant le fragment 1-107 de ExoS fusionné à la GFP pour tester l'expression de cette proteine fusion dans la ligne celluline CHO.

Le Demandeur a découvert que la sécrétion de protéines et l'injection de ces mêmes protéines dans une cellule hôte étaient possibles en mettant en oeuvre des séquences de ExoS de *P*. *aeruginosa* encore plus courtes, respectivement les 48 (SEQ ID 4) à 96 (SEQ ID 6), avantageusement les 54 (SEQ ID 5) premiers acides aminés N-terminaux de ExoS, et toute séquence identique à au moins 70%, avantageusement 75 % telle que par exemple la séquence d'acides aminés correspondante de ExoT. En outre, le Demandeur a démontré que la sécrétion et l'injection étaient plus efficaces lorsqu'on utilisait des séquences plus courtes. De ces résultats il découle donc que la séquence nucléotidique de ExoS peut être avantageusement modifiée pour coder au minimum les 48 AA N-terminaux de ExoS, au maximum les 96 premiers AA N-terminaux de ExoS de sorte que les souches de *P*. *aeruginosa* transformées avec ladite séquence nucléotidique modifiée peuvent être utilisées, soit pour l'injection de grosses protéines dans le cytosol d'une cellule hôte, soit pour la sécrétion, dans le milieu de culture, de protéines en vue de leur production.

En conséquence, l'invention concerne tout d'abord un vecteur d'expression d'une protéine chimère dont l'extrémité N-terminale correspond à la fusion entre l'extrémité N-terminale de ExoS de *P. aeruginosa* et une séquence d'acides aminés d'intérêt, l'extrémité N-terminale de ExoS correspondant à l'extrémité N-terminale de la protéine chimère, ledit vecteur étant caractérisé en ce qu'il contient de 5' en 3' :
- un promoteur,
- une séquence nucléotidique codant les 48 à 96 acides aminés de l'extrémité N-terminale de ExoS, avantageusement les 54 acides aminés ou toute séquence identique à au moins 70%,
- une séquence nucléotidique codant la séquence d'acides aminés d'intérêt.

Dans la suite de la description, et dans les revendications, par l'expression "séquence d'acides aminés d'intérêt", on désigne toute séquence d'acides aminés comprenant au moins 8 acides aminés constituant un peptide, un polypeptide ou encore une protéine naturelle ou synthétique présentant une activité biologique d'intérêt industriel pharmaceutique (thérapeutique, diagnostic) ou agroalimentaire. A titre d'exemple, de telles séquences peuvent correspondre à des séquences codant des antigènes de tumeur, des antigènes d'agents pathogènes, des protéines à activité antitumorale, anti-inflammatoire, anti-angiogénèse, anti-oxydante, des anticorps, épitopes, cytokines, récepteur de cytokine, facteur de transcription, facteur de signalisation intracellulaire, enzymes ou élément d'un complexe enzymatique.

De même, par l'expression "séquence identique à au moins 70 %", on désigne une séquence contenant au moins 70 %, avantageusement 75 % d'AA identiques aux 48, 96 ou 54 AA de ExoS. On a en effet constaté que la séquence nucléotidique de ExoT était identique à 75% à la séquence nucléotidique de ExoS, ce pourcentage étant obtenu par le programme LFASTA précité. En outre, cette identité de structure est confirmée par l'activité sécrétrice de ExoT qui, comme il sera vu ultérieurement, est du même niveau que celle de ExoS.

En d'autres termes, la séquence nucléotidique du vecteur d'expression contient deux séquences nucléotidiques fusionnées, respectivement une première séquence codant une séquence constituée des 48 à 96, avantageusement 54 premiers acides aminés N terminaux de ExoS et une seconde séquence codant la séquence d'acides aminés d'intérêt.

La fusion des deux séquences est obtenue par la mise en oeuvre de techniques connues de l'homme du métier et plus particulièrement décrite dans les ouvrages SAMBROOK J. **[4]**.

Selon une autre caractéristique, le promoteur peut être un promoteur de SSTT ou tout autre promoteur inductible ou non.

Le vecteur peut, par ailleurs, comprendre d'autres éléments tels qu'une séquence terminale 3' (incluant un codon stop) ou un gène de résistance à un antibiotique.

Le vecteur d'expression est construit selon des techniques parfaitement connues de l'homme du métier, en particulier par ligation de la séquence nucléotidique codant la séquence d'AA d'intérêt dans un vecteur de clonage codant l'extrémité N-terminale de ExoS, plus précisément les 48 à 96 AA, avantageusement 54 AA de l'extrémité N-terminale de ExoS.

En conséquence, l'invention a également pour objet un vecteur de clonage comprenant de 5' en 3' :
- un promoteur,
- une séquence nucléotidique codant les 48 à 96 acides aminés, avantageusement 54 premiers acides aminés de l'extrémité N-terminale de ExoS ou toute séquence identique à au moins 70 %.

Le vecteur de clonage peut en outre contenir d'autres éléments tels qu'une séquence nucléotidique codant pour un site de coupure permettant d'élimination de l'extrémité N-terminale de ExoS dans la protéine chimère, ou pour au moins un site de clonage permettant le clonage de toute protéine d'intérêt.

Les protéines chimères dont l'extrémité N-terminale est constituée des 48 à 96 AA, avantageusement 54 premiers AA de l'extrémité N-terminale de ExoS ou toute séquence identique à au moins 70% de *P. aeruginosa* font également partie de l'invention.

L'invention a également pour objet le gène codant pour la protéine chimère précitée.

L'invention concerne également une souche isolée de *P*. *aeruginosa,* transformée avec le vecteur d'expression ci-avant décrit, cette souche pouvant être utilisée soit pour la production de la protéine chimère dans le surnageant de culture, soit, après contact avec une cellule cible, pour le transfert de la protéine chimère dans ladite cellule cible.

Bien entendu, toutes les techniques de transformation connues de l'homme du métier peuvent être mises en oeuvre telles que électroporation, conjugaison triparentale, transfection par bactériophage...

Par ailleurs, le Demandeur a cherché à améliorer la sécrétion et le transfert de la protéine chimère en réduisant la toxicité des souches, par modifications d'au moins un gène codant les protéines ExoS, ExoT, ExoU ou ExoY de telle sorte à ce que ExoS et/ou ExoT soient incompétents pour leur production ou leur sécrétion par le système SSTT. En pratique, les modifications consistent en des mutations ou délétions.

Dans un mode de réalisation particulier, la souche sur laquelle est effectuée la transformation correspond à la souche CHA-003 déposée à la CNCM *(INSTITUT PASTEUR, 25 rue du Docteur Roux, F-75724 Paris cedex 15)* sous le numéro I-3090 le 17 septembre 2003, issue de la souche CHA **[3]** et dans laquelle les gènes codant l'exotoxine S (*exoS*) et l'exotoxine T (*exoT*) ont été inactivés. Cette souche possède les avantages suivants par rapport à la souche parentale CHA : une toxicité réduite à 20% de celle de CHA, une performance 10 fois supérieure de sécrétion et/ou injection.

L'invention concerne donc également une souche isolée de *Pseudomonas,* avantageusement *P. aeruginosa,* dont au moins un gène codant les protéines ExoS, ExoT, ExoU ou ExoY est modifié par délétion ou mutation. Dans un mode de réalisation particulier, la souche de *Pseudomonas* mutée correspond à la souche CHA-003 précitée.

Comme déjà dit, la fonction naturelle connue à ce jour des SSTT réside dans l'injection de protéines dans le cytosol de cellules eucaryotes.

L'invention concerne donc également un procédé d'injection *in vivo, ex vivo* ou *in vitro* dans une cellule eucaryote d'une protéine chimère dont l'extrémité N-terminale correspond à la fusion entre l'extrémité N-terminale de ExoS de *Pseudomonas aeruginosa* et une séquence d'acides aminés d'intérêt, l'extrémité N-terminale de ExoS correspondant à l'extrémité N-terminale de la protéine chimère, consistant :
- à construire le vecteur d'expression ci-avant décrit,
- puis à transformer une souche bactérienne avec ledit vecteur d'expression,
- puis à mettre ensuite en contact la souche transformée avec la cellule eucaryote.

De préférence, la souche utilisée pour la transformation est une souche de *Pseudomonas,* avantageusement *Pseudomonas aeruginosa,* dont au moins un gène codant les protéines ExoS, ExoT, ExoU ou ExoY, avantageusement *exoS* et *exoT* sont inactivés.

Dans un mode de réalisation particulier, la souche est la souche CHA-003.

Dans la suite de la description et dans les revendications, par «cellules eucaryotes », on désigne des cellules eucaryotes humaines ou animales, utilisées à des fins de recherche, de diagnostic ou de thérapie, *in vitro, ex vivo* ou *in vivo.*

Une autre application des SSTT qui n'est pas décrite dans l'art antérieur, consiste à utiliser ledit système pour la production par la bactérie de protéines chimères dans le surnageant de la culture.

En conséquence, l'invention a également pour objet un procédé de production d'une protéine chimère dont l'extrémité N-terminale correspond à la fusion entre l'extrémité N-terminale de ExoS de Pseudomonas *aeruginosa* et une séquence d'acides aminés d'intérêt, l'extrémité N-terminale de ExoS correspondant à l'extrémité N-terminale de la protéine chimère, ledit procédé consistant :
- à construire le vecteur d'expression ci-avant décrit,
- puis à transformer une souche bactérienne avec ledit vecteur d'expression,
- à induire ensuite la sécrétion de la protéine chimère,
- puis à récupérer le surnageant de culture.

De même que précédemment, la souche utilisée pour la transformation est, de préférence, une souche de *Pseudomonas,* avantageusement *Pseudonaonas aeruginosa,* dont au moins un gène codant les protéines ExoS, ExoT, ExoU ou ExoY, avantageusement ExoS et ExoT sont inactivés.

Dans un mode de réalisation particulier, la souche est la souche CHA-003.

L'induction de la sécrétion de la protéine chimère peut être obtenue par déplétion en Ca²⁺ ou par toute autre méthode activant le SSTT : sérum de veau foetal, sérum humain ou tout autre sérum, rouge Congo, ou tout autre milieu.

En pratique, la culture est poursuivie jusqu'à production optimale de la protéine chimère.

Enfin, le surnageant est récupéré par toute méthode connue de l'homme du métier telles que par exemple centrifugation, filtration et méthodes dérivées. La protéine d'intérêt peut être ensuite purifiée en utilisant toute technique de purification de protéine connue de l'homme de métier, par exemple : chromatographie d'affinité, d'échange d'ion, d'exclusion, immuno-précipitation etc.

L'invention et les avantages qui en découlent ressortiront mieux des exemples de réalisation suivants.

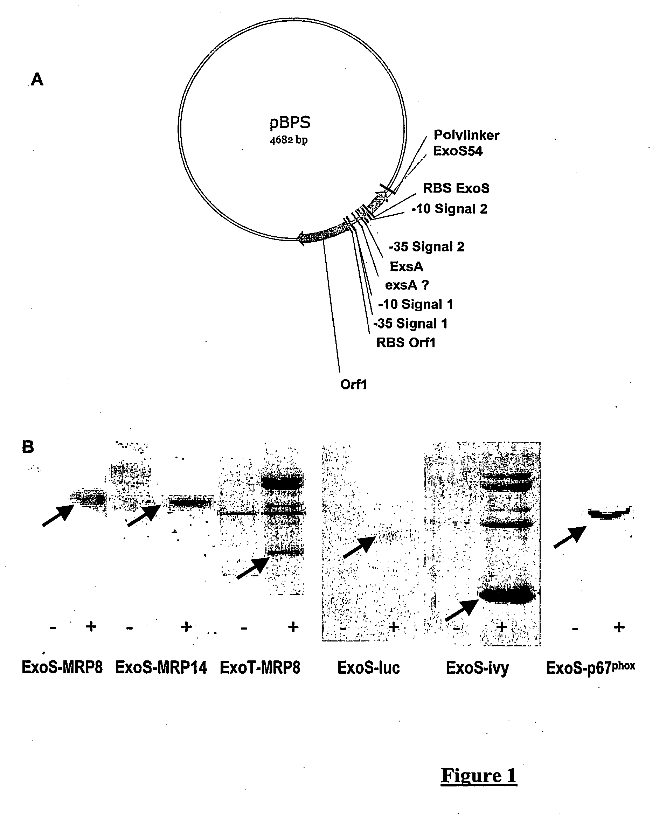

**Figure 1** **:** A) Le plasmide pBPS54 construit par insertion dans le plasmide pUCP20 **[2]** des séquences codant pour les 54 premiers acides aminés N-terminaux d'ExoS et la chaperone Orf1, tout en préservant le polylinker pour faciliter les clonages. B) Sécrétion des protéines de fusion avec (+) et sans (-) induction du SSTT.

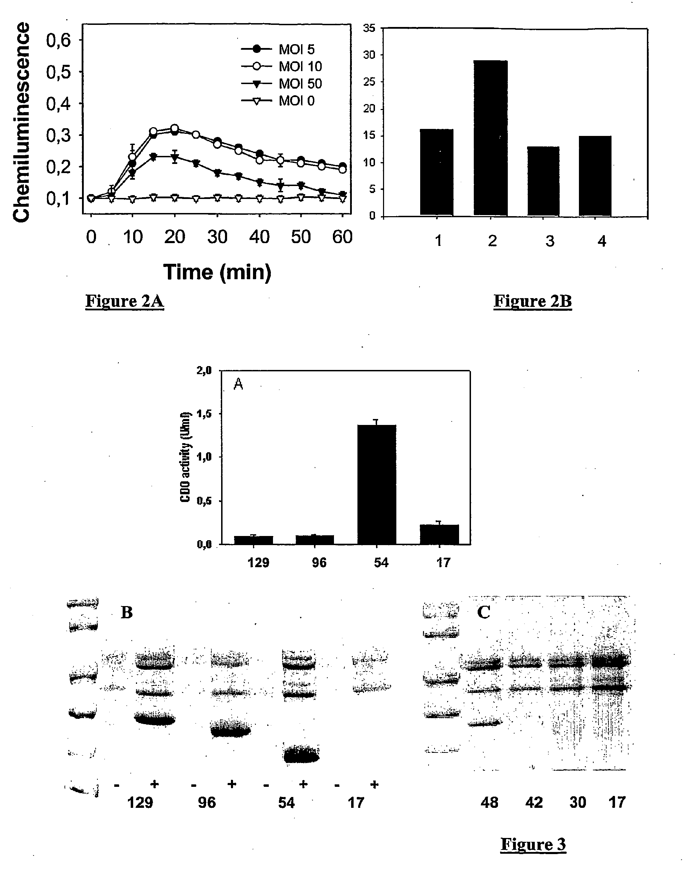

**Figure 2** **: A)** Mesure de la production d'anion superoxyde par la lignée lymphocytaire B GZ (déficiente en p67^{phox} en présence ou non du plasmide contenant la fusion ExoS54-p67^{phox}. **B)** Mesure de la production d'anion superoxyde par une lignée lymphocytaire normale avec 1) le plasmide contrôle, 2) un plasmide exprimant ExoS54-MRP8, 3) un plasmide exprimant ExoS54-MRP14, 4) les deux plasmides.
**Figure 3** **: A)** Activité CDO mesurée dans les surnageants obtenus après culture de bactéries en condition d'induction de la sécrétion par les fusions ExoS-XylE avec les 129 (SEQ ID7), 96, 54 et 17 premiers acides aminés d'ExoS. **B)** et **C)** Les protéines de fusion ExoS-MRP8 sécrétées dans le surnageant sont séparées par électrophorèse en gel de polyacrylamide en présence de SDS et colorées au bleu de Coomasie. Le panneau A représente les fusions ExoS-MRP8 avec les 129, 96, 54 et 17 premiers acides aminés d'ExoS en condition d'induction de la sécrétion (+) et en conditions n'induisant pas la sécrétion (-) du SSTT. Le panneau C représente les fusions ExoS-MRP8 avec les 48 (SEQ ID4), 42 (SEQ ID3), 30 (SEQ ID2) et 17 (SEQ ID1) premiers acides aminés d'ExoS en condition d'induction de la sécrétion du SSTT.

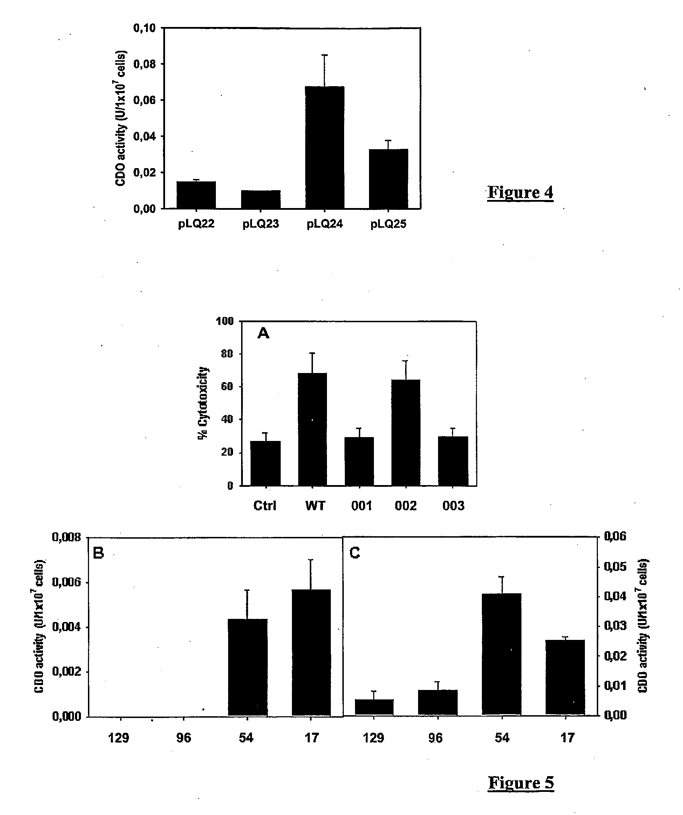

**Figure 4** **:** Activité CDO mesurée dans les cytosols des cellules PLB985 après une heure de co-culture bactérie/cellule dans un rapport de 10 à 1. Les bactéries contiennent les plasmides codant pour les fusions de XylE avec les 129 (pLQ22) 96 (pLQ23) 54 (pLQ24) et 17 (pLQ25) premiers acides aminés d'ExoS.
**Figure 5** **: A)** Toxicité de la souche sauvage (WT) et des mutants délétés dans les toxines du SSTT CHA-001 délété pour *exoS,* CHA-002 délété pour *exoT* et CHA-003 délété pour *exoS* et *exoT.* **B)** et **C)** Quantification par XylE de l'injection de la souche sauvage CHA (B) et CHA-003 délétée en ExoS et ExoT (C). Celle-ci injecte 10 fois plus que la souche sauvage.

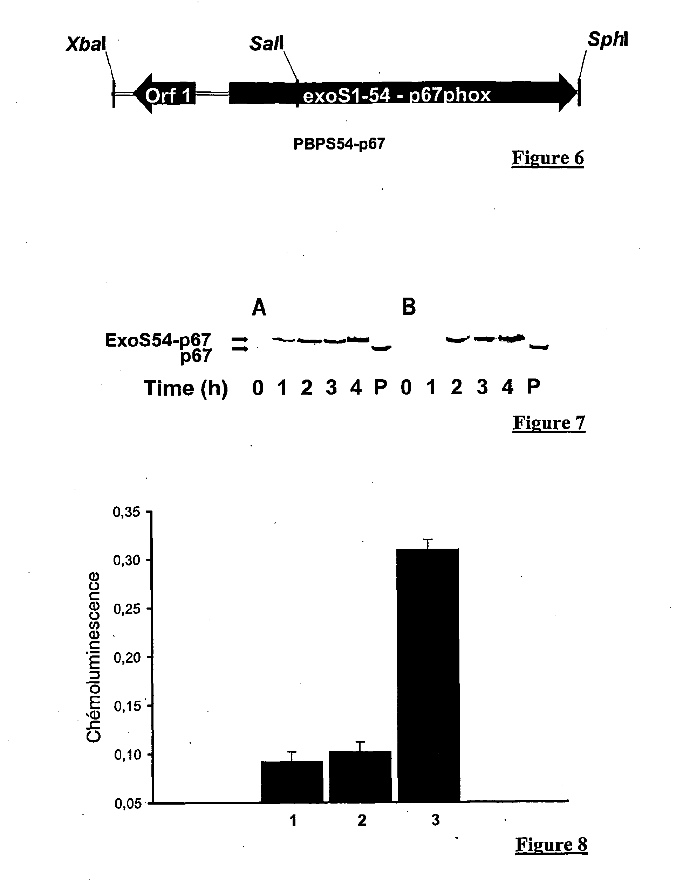

**Figure 6** **:** Le plasmide pBPS54-p67 code une protéine hybride comprenant le domaine N-terminal de la protéine ExoS fusionnée avec le facteur cytosolique de la NADPH oxydase, p67^{phox}. La cassette de clonage *Sal*I*-Sph*I permet l'insertion en phase de la protéine d'intérêt.
**Figure 7** **:** Biosynthèse et sécrétion d'ExoS-p67^{phox} par CHA-p31 après stimulation par chélation du calcium. La protéine hybride ExoS-p67^{phox} est détectée par Western blot avec un anticorps dirigé contre l'extrémité C-terminale de p67^{phox} soit dans le culot bactérien lysé aux ultrasons (A) soit dans le milieu de culture (B). P correspond à la protéine p67^{phox} native, purifiée à partir des phagocytes.
**Figure 8** **:** Reconstitution fonctionnelle de la NADPH oxydase dans la lignée GZ de Lymphocytes B EBV déficiente en p67^{phox}. Production d'anion superoxyde à la 15^{éme} minute d'incubation par les lymphocytes déficients en p67^{phox}. 1) lymphocytes déficients en p67^{phox} seuls, 2) lymphocytes déficients en p67^{phox} en présence de la souche CHA, 3) lymphocytes déficients en p67^{phox} en présence de la souche CHA-003 contenant le plasmide pBPS54-p67 avec un rapport d'infection de 10.

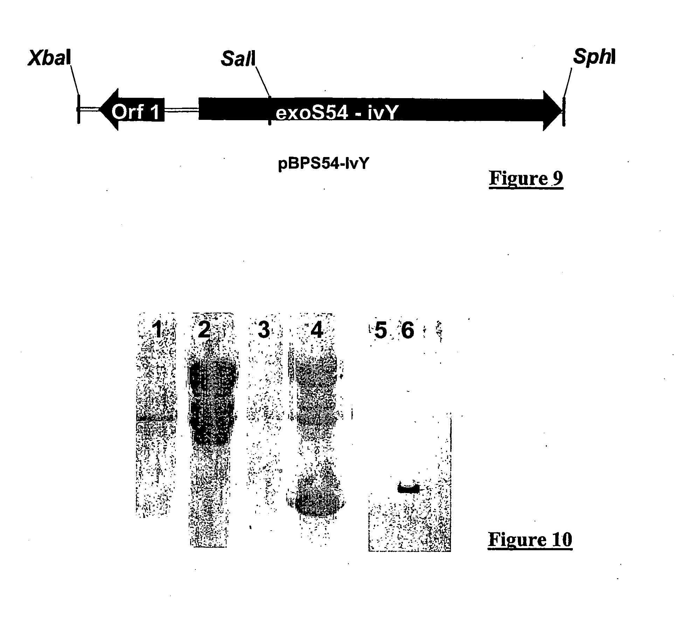

**Figure 9** **:** Le plasmide pBPS54-ivY code une protéine hybride comprenant le domaine N-terminal de la protéine ExoS fusionnée avec le facteur d'inhibition du lysozyme IVY de *P.aeruginosa.* La cassette de clonage *Sal*I*-Sph*I permet l'insertion en phase de la protéine d'intérêt.
**Figure 10** **:** Production de la protéine ExoS-IVY. 1 ml de surnageant avant ou après induction a été précipité par l'acide perchloracétique et analyse en gel de SDS-PAGE et révélé au bleu de Coomasie. 1/ souche CHA non induite, 2/ souche CHA induite, 3/souche CHA contenenant le plasmide pBPS54-ivY non induite, 4/ souche CHA contenant le plasmide pBPS54-ivy induite. La même protéine avec une étiquette poly-histidine est purifiée sur une colonne d'affinité de nickel immobilisé : 5/ lavage après fixation sur colonne IMAC par imidazol 10 mM, 6/ élution par l'imidazol 300 mM.

### Exemple 1 : Propriétés sécrétrices de ExoS et ExoT

Cet exemple démontre qu'une protéine d'intérêt peut être produite et sécrétée par *P.aeruginosa* lorsqu'elle est fusionnée avec les 54 premiers acides aminés d'ExoS ou d'ExoT. Les gènes codant pour les protéines d'intérêt, par exemple MRP8, MRP14, XylE, IVY, p67phox sont clonés en phase avec la séquence codant pour les toxines sécrétées par le SSTT, par exemple ExoS et ExoT. Cette séquence, optimalement codant les 54 premiers acides aminés, est placée sous le contrôle du promoteur naturel de la dite toxine. L'ensemble de la construction est porté par un plasmide qui se réplique dans *P.aeruginosa* (Figure 1A).

Le plasmide est introduit par électroporation dans une souche de *P.aeruginosa.* Les bactéries sont cultivées soit en l'absence d'induction du SSTT (milieu LB) soit en présence d'EGTA 5 mM, MgCl₂ 20 mM qui permettent l'induction de la synthèse des protéines et leur sécrétions dans le surnageant. L'analyse des surnageants de culture en l'absence ou en présence d'induction de la sécrétion est faite sur gel de polyacrylamide en conditions dénaturantes (SDS-PAGE) après précipitation des protéines de 1 ml de surnageant par l'acide perchlorique 10%. La révélation est faite par coloration au bleu de Coomassie pour MRP8, MRP14, IVY et au nitrate d'argent pour Luc et P67phox. On constate sur les pistes avec induction (notées + ) la présence d'une bande dont la migration correspond bien, à la taille de la protéine attendue (flèche). En terme de quantité, la meilleure sécrétion est obtenue pour la protéine Ivy fusionnée à ExoS-54 ce qui permet la production d'environ 10 microG de protéine par ml de surnageant. Pour la plupart des protéines testées nous obtenons des taux de production se situant entre 0.5 et 10 mg de protéine par litre de surnageant.

### Exemple 2 : Propriétés injectrices de ExoS

Cet exemple démontre qu'une protéine d'intérêt peut être produite et injectée dans une cellule d'intérêt par *P.aeruginosa* lorsqu'elle est fusionnée avec les 54 premiers acides aminés d'ExoS. Les gènes codant pour les protéines d'intérêt, par exemple P67phox, MRP8 et MRP14 sont clonés en phase avec la séquence codant pour les toxines sécrétées par le SSTT, par exemple ExoS et ExoT. Cette séquence, optimalement codant les 54 premiers acides aminés, est placée sous le contrôle du promoteur naturel de la dite toxine. L'ensemble de la construction est porté par un plasmide qui se réplique dans *P.aeruginosa.*

Le plasmide est introduit par électroporation dans une souche de *P.aeruginosa.* Les bactéries sont cultivées en milieu LB en l'absence d'induction du SSTT puis sont mises en présence de la cellule eucaryote cible (lymphocytes B pour p67phox). Le rapport bactérie / cellule eucaryote (MOI) est de 1 à 50, optimalement 10. Après une heure de co-culture, l'activité NADPH oxydase résultante de la transduction des protéines sus-décrites est mesurée par la production d'ions superoxyde sur les cellules entières, par chimioluminescence comme décrit dans l'article [3]. La figure 2A montre l'effet du rapport d'infection sur l'efficacité de l'injection. La cinétique de production de l'ion superoxyde par les lymphocytes B ainsi modifiés est en faveur d'un rapport d'infection situé entre 5 et 10. La plupart des expériences montrent que le rapport optimal d'infection est de 1 à 10 pour les différents types de lignées ainsi transformées (lymphocytes, polynucléaires neutrophiles, macrophages, lignée granuleuse PLB985, cellules dendritiques, ...).

L'exemple 2B montre que l'on peut utiliser ce procédé d'injection *in vitro* pour mesurer les effets respectifs et/ou concomitants de deux ou plus protéines d'intérêt. La barre 1 montre le niveau basal d'activité en unité relative de chémioluminescence lorsque des lymphocytes B normaux sont mis en présence de CHA-003 portant un plasmide contrôle ne contenant pas de gène d'intérêt. La barre 2 montre l'augmentation de l'activité NADPH oxydase lorsque la protéine ExoS54-MRP8 est injectée par la bactérie (MOI = 10) qui contient une construction codant pour une protéine de fusion ExoS54-MRP8. La barre 3 montre l'absence d'accroissement de l'activité basale de la NADPH oxydase lors de l'injection par la bactérie de la protéine de fusion ExoS54-MRP14. La barre 4 montre l'inhibition de l'accroissement de l'activité de la NADPH oxydase lorsque les cellules sont incubées en présence des deux souches précédemment décrites et permettant l'expression d'ExoS54-MRP8 et d'ExoS54-MRP14.

Ce système permet donc également de réaliser l'injection concomitante de une, deux ou plusieurs protéines.

### Exemple 3 : Amélioration de la sécrétion avec ExoS54

L'optimum de longueur de la séquence d'ExoS permettant la sécrétion a été déterminé en utilisant des constructions de longueur variables fusionnées avec la protéine XylE dont l'activité enzymatique catéchol dioxygénase a été mesurée dans le surnageant de culture **[4].**

La souche CHA-003 a été transformée par différents plasmides permettant les expressions des fusions ExoS-XylE contenant les 129 (SEQ ID 7), 96, 54 et 17 (SEQ ID1) premiers acides aminés d'ExoS. La diminution de 129 à 54 acides aminés d'ExoS permet une augmentation d'au moins 6 fois de l'activité CDO mesurée dans le surnageant de culture. Les mêmes résultats ont été obtenus avec la protéine MRP8. La figure 3B a été obtenue comme précédemment avec la protéine MRP8 à la place de XylE. Les bactéries transformées sont activées pour sécréter les protéines de fusion par déplétion en calcium pendant 3 heures. 1 ml de surnageant de culture est précipité à l'acide perchlorique pendant 1 heure comme décrit ci-dessus. Les protéines sont séparées sur un gel de polyacrylamide en présence de SDS et colorées au bleu de Coomasie. Pour MRP8 les fusions 129, 96 et 54 permettent une excellente sécrétion. La figure 3C montre cependant que la sécrétion de ExoS-MRP8 s'arrête dès lors que moins de 48 acides aminés de ExoS sont utilisés. Les exemples de XylE (3A) et de MRP8 (3B et C) ainsi que d'autres protéines testées montrent qu'une fusion avec les 54 premiers acides aminés d'ExoS est optimale pour la plupart des protéines.

### Exemple 4 : Amélioration de l'injection avec ExoS54

L'optimum de longueur de la séquence d'ExoS permettant l'injection dans les cellules eucaryotes a également été déterminé en utilisant des constructions de longueur variables fusionnées avec la protéine XylE dont l'activité enzymatique catéchol dioxygénase a été mesurée dans le cytosol des cellules PLB985 après une heure de co-culture bactérie/cellule dans un rapport de 10 à 1. Comme attendu, puisque la sécrétion est optimale avec les 54 premiers acides aminés d'ExoS, l'injection suit les mêmes règles (figure 4). La séquence ExoS54 peut donc être utilisée comme étiquette universelle permettant soit la sécrétion soit l'injection de protéines d'intérêt pour la recherche, le diagnostic ou la thérapeutique.

### Exemple 5 : Amélioration de la souche

L'amélioration de la souche a été initialement faite pour éviter les phénomènes de cytotoxicité lors de l'utilisation du SSTT à des fins d'injection de protéines dans des cellules de mammifère. C'est pourquoi le premier critère retenu a été la diminution de la toxicité naturelle de la souche CHA dû aux toxines ExoS et ExoT. Différents mutants d'inactivation ont été obtenus par invalidation des gènes selon les méthodes connues de l'homme de l'art **[5].** Ces méthodes consistent à insérer au sein du gène que l'on veut inactiver soit une délétion soit une interruption du cadre de lecture. Le gène ainsi modifié prend la place du gène sauvage lors d'une expérience de simple ou double recombinaison par toute méthode connue de l'homme de l'art. Les souches ainsi obtenues ne permettent plus l'expression des gènes ciblés. Nous avons obtenu à partir de la souche CHA (sauvage), CHA-001 délétée dans *exoS,* CHA-002 délétée dans *exoT* et CHA-003 délétée dans *exoS* et *ExoT.* Leur toxicité vis à vis de cellules cibles a été mesurée (figure 5). La toxicité de ces souches sur plusieurs cellules (lignée granulocytaire PLB985, polynucléaires neutrophiles humain, macrophages murins, et cellules dendritiques) ont été mesurées lors d'expériences de co-culture bactéries-cellules à différents rapports d'infection durant des périodes d'incubation de une à quatre heures, en mesurant l'activité lactate déhydrogénase libérée par la lyse des cellules sous l'action toxique de la bactérie. L'ensemble des résultats est compilé dans la figure 5A qui montre la très nette diminution de la toxicité qui devient comparable au bruit de fond lorsqu'on inactive *exoS* et *exoT.*

Puisque les toxines ExoS et ExoT sont aussi transportées par le SSTT, nous avons mesuré l'effet de cette inactivation pour l'amélioration de l'injection de protéines d'intérêt. La construction ExoS54-XylE a été introduite dans les différents mutants et les bactéries ont été mises en co-culture avec les cellules PLB985 à un rapport de 10 ce qui nous a permis de quantifier le gain de l'injection. Une injection dix fois supérieure est obtenue grâce à l'utilisation de la souche CHA-003 délétée en ExoS et ExoT.

### Exemple 6: Procédé d'injection

Il peut être intéressant à des fins de recherches, de diagnostique ou de thérapie d'injecter directement dans une population de cellules de mammifères, à l'aide du SSTT de *P.aeruginosa* des protéines d'intérêt. Le procédé d'injection décrit ci-dessous peut s'appliquer à des cellules en cultures ou dans un organisme entier vivant lorsque la toxicité due à l'injection de *P.aeruginosa* peut-être tolérée. Ce procédé s'applique à tout type de protéine. Des optimisations doivent éventuellement être apportées à chacunes des étapes du procédé en fonction du type de protéines et/ou du type de cellules que l'on veut transformer. Les principales optimisations peuvent porter sur : le rapport d'infection à utiliser, le temps de co-culture, le milieu utilisé lors de la coculture. Le procédé peut se décrire schématiquement de la manière suivante pour des cellules en cultures.

Le gène codant la protéine d'intérêt est cloné en phase avec la partie N-terminale de ExoS ou ExoT, optimalement avec les 54 premiers acides aminés de ExoS. On utilise tout type de plasmide se repliquant dans *P.aeruginosa,* permettant l'expression de telles fusions comme par exemple celui décrit en exemple 1.

Le plasmide est introduit par électroporation ou tout autre méthode connue de l'homme de l'art, dans une souche de *P.aeruginosa,* optimalement la souche CHA-003. Les bactéries sont cultivées en milieu LB en l'absence d'induction du SSTT jusqu'au milieu de la phase exponentielle de croissance. Les bactéries sont alors récupérées par centrifugation, lavées et re-suspendues dans le milieu de culture utilisé pour les cellules de mammifères que l'on veut transformer. Les bactéries sont mises en présence des cellules cibles. Le rapport bactérie / cellule eucaryote (MOI) est de 1 à 50, optimalement 10. Après une heure de co-culture on peut éliminer les bactéries par lavage et addition d'antibiotiques dans le milieu de culture. Le phénotype produit peut alors être observé ou bien les cellules mises en culture si le phénotype produit nécessite une durée d'action plus longue.

### Exemple : injection intracytosolique d'une protéine humaine dans des lymphocytes B humains.

A l'aide du SSTT de *P.aeruginosa,* nous avons réalisé l'injection intracytosolique d'une protéine active capable de reconstituer un complexe enzymatique d'assemblage hautement régulé, la NADPH oxydase, dans des cellules issues de patients porteur du déficit en cet enzyme. La NADPH oxydase produit les radicaux superoxydes qui, lors de l'explosion métabolique phagocytaire, vont permettre la destruction des microorganismes pathogènes. Cette enzyme est également présente dans d'autres cellules dont les lymphocytes B immortalisés par le virus d'Epstein-Barr (Lymphocytes B EBV). L'activation de la NADPH oxydase implique l'assemblage de 4 protéines cytosoliques (p47^{phox}, p67^{phox}, p40^{phox} et une petite protéine G monomérique Rac1 ou Rac2) avec une glycoprotéine transmembranaire hétérodimérique, le flavocytochrome b₅₅₈ composé de deux sous-unités, p22^{phox} et gp91^{phox}. Des anomalies moléculaires dans les gènes codant pour p47^{phox}, p67^{phox}, p22^{phox} ou gp91^{phox} entraînent une maladie sévère appelée la granulomatose septique chronique (CGD) caractérisée sur le plan biochimique par l'absence de production d'anion superoxyde et sur le plan clinique par des infections bactériennes sévères et à répétition qui, malgré les antibiotiques, mettent en jeu le pronostic vital des patients. Un patient dont les phagocytes et les Lymphocytes B EBV sont incapables de générer des ions superoxydes a été pris comme modèle. Ces cellules n'expriment en effet ni l'ARNm de p67^{phox}, ni cette protéine. Puisque p67^{phox} est un facteur cytosolique, nous avons pris ce modèle pour analyser et valider le transport vectorialisé des protéines dans le cytosol et la capacité des protéines ainsi transférées d'être activées et insérées dans un complexe enzymatique multimoléculaire dont la régulation de l'activation met en jeu des interactions moléculaires hautement sophistiquées.

Les vecteurs ont été développés à partir de la bactérie *P. aeruginosa* (souche CHA-003). Nous avons construit le plasmide pBPS54-p67 qui code pour une protéine hybride, ExoS-p67^{phox}, formée des 54 premiers acides aminés N-terminaux d'ExoS fusionnés avec l'acide aminé N-terminal de p67^{phox}. Cette construction est placée devant le promoteur naturel de ExoS et l'ensemble est porté par le plasmide pUCp20. La chaperonne spécifique d'ExoS, Orf1, est incluse dans son orientation originale pour optimiser le transfert cytosolique de la protéine chimérique (Fig. 6).

La souche CHA-003 de *P. aeruginosa* contenant le plasmide pBPS54-p67 synthétise et sécrète la protéine hybride après stimulation par la chélation du calcium (Fig. 7A et B), La protéine hybride est donc synthétisée et sécrétée dans les mêmes conditions que la protéine ExoS native.

Par western blot à l'aide d'anticorps anti p67^{phox}, nous avons vérifié que la protéine hybride ExoS-p67^{phox} est également injectée dans le cytosol des Lymphocytes B EBV. La concentration cytosolique qu'elle y atteint est comparable à celle de la protéine p67^{phox} endogène. Cette injection cytosolique est bien sous la dépendance du système de sécrétion de type III.

Une expérience de reconstitution *in vitro* de l'activité NADPH oxydase indique que la protéine hybride garde l'ensemble de ses domaines fonctionnels accessible. A l'intérieur des cellules, de nombreux autres facteurs sont impliqués. En effet, le trafic cellulaire des protéines est initié physiologiquement lors de leur synthèse dans le réticulum endoplasmique et continue lors de la maturation de la protéine à travers les différentes parties de l'appareil de Golgi, pour se terminer à leur site d'action qui peut être le noyau, l'une des organelles intracellulaire, ou l'un des compartiments cytosolique. Un adressage complexe, dont la compréhension n'est que partielle, permet donc *in fine* à la protéine mature d'être localisée dans le compartiment sub-cellulaire où elle doit jouer son rôle. Les capacités d'auto-adressage d'une molécule hybride comme ExoS-p67^{phox} dans le compartiment sous membranaire où se situe vraisemblablement l'assemblage final de la NADPH oxydase ont donc été testées *ex vivo* sur des Lymphocytes B EBV normaux et sur des Lymphocytes B EBV déficients en p67^{phox}, la lignée cellulaire GZ. Lorsque la lignée GZ est incubée avec un nombre de bactérie par cellule (MOI) de 5, 10 ou 50, il y a une restauration de 40 à 50% de l'activité du complexe enzymatique (Figure 8).

### Exemple 7 : procédé de production

La plupart des protéines recombinantes peuvent être obtenues à partir de bactéries telles que *Escherichia coli,* ou en cellules eucaryote (levures, cellules d'insecte ou cellules de mammifères). Néanmoins l'expérience montre que nombre de protéines ne peuvent être obtenues par ces hôtes classiques de production soit parce que leur expression est toxique pour l'hôte de production, soit en raison de dégradation due au mauvais repliement de la protéine à cause de conditions défavorables dans l'hôte de production. L'utilisation du système de sécrétion de type III de *P.aeruginosa* permet l'expression et la sécrétion directe des protéines dans le surnageant de culture. Ce système est donc bien adapté à la production des protéines recombinantes ayant soit une toxicité intracellulaire soit un défaut de production ou de repliement à l'intérieur des bactéries. Nous avons démontré dans les différents exemples de ce brevet que les taux de production peuvent atteindre 10 mg par litre de culture. Par ailleurs, surtout lorsqu'on utilise la souche CHA-003 qui ne sécrète pas les exotoxines S et T la protéine produite devient majoritaire dans le surnageant de culture ce qui facilite les étapes ultérieures de purification.

L'exemple donné est celui de la protéine Ivy qui code pour un facteur d'inhibition du lysozyme chez les bactéries. Cette protéine est obtenue très difficilement sous forme recombinante dans *E.coli.* Le gène codant la protéine Ivy de *P.aeruginosa* a été amplifié par PCR à l'aide d'amorces contenant les sites de clonage en phase avec les 54 premiers amino-acides de ExoS.

Le plasmide pBPS54-Ivy a été introduit par électroporation dans la souche CHA-003. Les bactéries ont été cultivées une nuit à 37°C puis diluées le matin dans du milieu LB frais en présence ou non d'EGTA qui permet la déplétion calcique et donc l'induction du SSTT.

La bande montrée avec une flèche sur la figure 10 piste 4 migre à la taille de 22 kDa et correspond à une quantité de l'ordre 10 µg de protéine par ml de surnageant, soit 10 mg/l. Lorsque la protéine porte une étiquette poly-histidine sa purification par chromatographie d'affinité sur nickel immobilisé (IMAC) peut être effectuée directement à partir du surnageant de culture après induction et dialyse pour éliminer l'EGTA. La figure 10 piste 6 montre bien la présence d'une unique bande correspondant à la protéine hybride purifiée dans l'éluat de la colonne IMAC.

### RÉFÉRENCES

1. CORNELIS et Al. (1997) Mol. Microbiol. 23(5).
2. SORY et Al. (1995), Proc. Natl. Acad. Sci. USA 92:11998-12002.
3. POLACK, B., VERGNAUD, S., PACLET, M. H., LAMOTTE, D., TOUSSAINT, B., MOREL, F.: Protein delivery by Pseudomonas type III secretion system: Ex vivo complementation of p67phox deficient chronic granulomatous disease. Biochem. Biophys. Res. Commun. 275, 854-858, 2000**.**
4. SAMBROOK J., Russel DW, Molecular cloning, a laboratory manual, 3rd edition, Cold Spring Harbor Laboratry Pres, CSH 2001**.**
5. DELIC-ATTREE, L, TOUSSAINT, B., VIGNAIS, P. M.: Cloning and sequence analyses of the genes coding for the integration host factor (IHF) and HU proteins of Pseudomonas aeruginosa. Gene 154, 61-64, 1995.
6. WEST, S. E. H., SCHWEIZER, H. P., DALL, C., SAMPLE, A. K., RUNYEN-JANECKY, L. J.: Construction of improved Escherichia-Pseudomonas shuttle vectors derived from pUC18/19 and sequence of the region required for their replication in Pseudomonas aeruginosa. Gene 128, 81-86, 1994**.**
7. KONYECSNI, W. M., DERETIC, V.: Broad-host-range plasmid and M13 bacteriophage-derived vectors for promoter analysis in Escherichia coli and Pseudomonas aeruginosa. Gene 74, 375-386, 1988.
8. SCHWEIZER, H. P., HOANG, T. T.: An improved system for gene replacement and xylE fusion analysis in Pseudomonas aeruginosa. Gene 158, 15-22, 1995.

## Revendications

1. Vecteur d'expression d'une protéine chimère dont l'extrémité N-terminale correspond à la fusion entre l'extrémité N-terminale de ExoS de *Pseudomonas aeruginosa* et une séquence d'acides aminés d'intérêt, l'extrémité N-terminale de ExoS correspondant à l'extrémité N-terminale de la protéine chimère, ledit vecteur étant **caractérisé en ce qu'**il contient de 5' en 3' :
- un promoteur,
- une séquence nucléotidique codant pour les 54 (SEQ ID 5) premiers acides aminés de l'extrémité N-terminale de ExoS, ou pour toute séquence identique à au moins 75 % à SEQ ID 5;
- une séquence nucléotidique codant la séquence d'acides aminés d'intérêt.

2. Vecteur selon la revendication 1, **caractérisé en ce que** la séquence nucléotidique code pour les 54 premiers acides aminés de l'extrémité N-terminale de ExoT de *P. aeruginosa.*

3. Vecteur de clonage comprenant de 5' en 3' :
- un promoteur,
- une séquence nucléotidique codant pour les 54 (SEQ ID 5) premiers acides aminés de l'extrémité N-terminale de ExoS, ou pour toute séquence identique à au moins 75 % à SEQ ID 5.

4. Vecteur de clonage selon la revendication 3, **caractérisé en ce que** la séquence nucléotidique code pour les 54 premiers acides aminés de l'extrémité N-terminale de ExoT de *Pseudomonas aeruginosa.*

5. Protéine chimère dont l'extrémité N-terminale est constituée des 54 (SEQ ID 5) premiers acides aminés de l'extrémité N-terminale de ExoS, ou de toute séquence identique à au moins 75 % à SEQ ID 5.

6. Protéine chimère selon la revendication 5, **caractérisée en ce que** son extrémité N-terminale est constituée des 54 premiers acides aminés de l'extrémité N-terminale de ExoT de *P. aeruginosa.*

7. Gène codant pour la protéine chimère objet de l'une des revendications 5 ou 6.

8. Souche isolée de *Pseudomonas aeruginosa* transformée avec le vecteur d'expression objet de l'une des revendications 1 à 4.

9. Souche selon la revendication 8, **caractérisée en ce que** la souche est modifiée par délétion ou mutation d'au moins un gène sélectionné parmi les gènes codant les protéines ExoS, ExoT, ExoU et ExoY.

10. Souche selon la revendication 9, **caractérisée en ce que** la souche a été déposée à la CNCM (Institut Pasteur de Paris) sous le numéro I-3090.

11. Procédé d'injection *ex vivo* ou *in vitro* dans une cellule eucaryote d'une protéine chimère selon la revendication 5 ou 6, ou codée par un vecteur selon l'une des revendications 1 à 4, comprenant la mise en contact d'une souche selon l'une des revendications 8 à 10 avec la cellule eucaryote.

12. Procédé de production d'une protéine chimère selon la revendication 5 ou 6, ou codée par un vecteur selon l'une des revendications 1 à 4, comprenant les étapes suivantes :
- cultiver une souche selon l'une des revendications 8 à 10,
- induire la sécrétion de la protéine chimère,
- récupérer le surnageant de culture.

13. Souche selon l'une des revendications 8 à 10 pour son utilisation en thérapie.

## Claims

1. Expression vector of a chimeric protein whose N-terminal end corresponds to the fusion between the N-terminal end of ExoS of *Pseudomonas aeruginosa* and an amino acid sequence of interest, the N-terminal end of ExoS corresponding to the N-terminal end of the chimeric protein, the said vector being **characterised in that** it contains the following from 5' to 3':
- a promoter;
- a nucleotide sequence encoding the first 54 (SEQ ID 5) amino acids of the N-terminal end of ExoS, or any sequence that is at least 75 % identical to SEQ ID 5;
- a nucleotide sequence encoding the amino acid sequence of interest.

2. Vector as claimed in claim 1, **characterised in that** the nucleotide sequence encodes the first 54 amino acids of the N-terminal end of ExoT of *P*. *aeruginosa.*

3. Cloning vector comprising from 5' to 3':
- a promoter;
- a nucleotide sequence encoding the first 54 (SEQ ID 5) amino acids of the N-terminal end of ExoS, or any sequence that is at least 75 % identical to SEQ ID 5.

4. Cloning vector as claimed in claim 3, **characterised in that** the nucleotide sequence encodes the first 54 amino acids of the N-terminal end of ExoT of *P*. *aeruginosa.*

5. Chimeric protein whose N-terminal end corresponds to the first 54 (SEQ ID 5) amino acids of the N-terminal end of ExoS, or to any sequence that is at least 75 % identical to SEQ ID 5.

6. Chimeric protein as claimed in claim 5, **characterised in that** its N-terminal end corresponds to the first 54 amino acids of the N-terminal end of ExoT of *P*. *aeruginosa.*

7. Gene encoding the chimeric protein, object of the claims 5 and 6.

8. Isolated strain of *Pseudomonas aeruginosa* transformed with the expression vector, object of the claims 1 and 2.

9. Strain as claimed in claim 8, **characterised in that** the transformation is carried out on a strain whose at least one gene encoding the ExoS, ExoT, ExoU or ExoY proteins is modified by deletion or mutation.

10. Strain as claimed in claim 9, **characterised in that** the strain has been deposited at CNCM (Institut Pasteur - Paris) under number I-3090.

11. *Ex vivo* or *in vitro* injection procedure into a eukaryotic cell of a chimeric protein object of the claims 5 and 6, or encoded by a vector object of the claims 1 to 4, comprising bringing into contact the strain object of the claims 8 to 10 with the eukaryotic cell.

12. Production procedure of a chimeric protein object of the claims 5 and 6, or encoded by a vector object of the claims 1 to 4, comprising the following steps:
- growth of the strain object of the claims 8 to 10;
- subsequent induction of secretion of the chimeric protein;
- then recovery of the culture supernatant.

13. Strain as claimed in claims 8 to 10 for its use in therapy.

## Patentansprüche

1. Vektor zur Expression eines chimären Proteins, dessen N-terminales Ende der Fusion zwischen dem N-terminalen Ende von ExoS von *Pseudomonas aeruginosa* und einer Aminosäurensequenz von Interesse entspricht, wobei das N-terminale Ende von ExoS dem N-terminalen Ende des chimären Proteins entspricht, wobei dieser Vektor **dadurch gekennzeichnet ist, dass** er in 5'-nach-3'-Richtung enthält:
- einen Promotor,
- eine Nukleotidsequenz, die für die 54 (SEQ ID 5) ersten Aminosäuren des N-terminalen Endes von ExoS oder für jede zu mindestens 75% mit SEQ ID 5 identische Sequenz kodiert;
- eine Nukleotidsequenz, die die Aminosäurensequenz von Interesse kodiert.

2. Vektor nach Anspruch 1, **dadurch gekennzeichnet, dass** die Nukleotidsequenz für die ersten 54 Aminosäuren des N-terminalen Endes von ExoT von *P*. *aeruginosa* kodiert.

3. Klonierungsvektor, umfassend in 5'-nach-3'-Richtung:
- einen Promotor,
- eine Nukleotidsequenz, die für die ersten 54 (SEQ ID 5) Aminosäuren des N-terminalen Endes von ExoS oder für jede zu mindestens 75% mit SEQ ID 5 identische Sequenz kodiert.

4. Klonierungsvektor nach Anspruch 3, **dadurch gekennzeichnet, dass** die Nukleotidsequenz für die ersten 54 Aminosäuren des N-terminalen Endes von ExoT von *Pseudomonas aeruginosa* kodiert.

5. Chimäres Protein, dessen N-terminales Ende aus den ersten 54 (SEQ ID 5) Aminosäuren des N-terminalen Endes von ExoS oder jeder zu mindestens 75% mit SEQ ID 5 identischen Sequenz besteht.

6. Chimäres Protein nach Anspruch 5, **dadurch gekennzeichnet, dass** sein N-terminales Ende aus den ersten 54 Aminosäuren des N-terminalen Endes von ExoT von *P. aeruginosa* besteht.

7. Gen, das für das chimäre Protein kodiert, das Gegenstand eines der Ansprüche 5 oder 6 ist.

8. Isolierter Stamm von *Pseudomonas aeruginosa,* der mit dem Expressionsvektor, der Gegenstand eines der Ansprüche 1 bis 4 ist, transformiert wurde.

9. Stamm nach Anspruch 8, **dadurch gekennzeichnet, dass** der Stamm durch Deletion oder Mutation mindestens eines Gens modifiziert ist, das aus den die Proteine ExoS, ExoT, ExoU und ExoY kodierenden Genen selektiert ist.

10. Stamm nach Anspruch 9, **dadurch gekennzeichnet, dass** der Stamm bei CNCM (Institut Pasteur Paris) unter der Nummer I-3090 hinterlegt wurde.

11. Verfahren zur *ex-vivo-* oder in-vitro-Injektion eines chimären Proteins nach Anspruch 5 oder 6 oder eines durch einen Vektor nach einem der Ansprüche 1 bis 4 kodierten chimären Proteins in eine eukaryotische Zelle, umfassend das In-Kontakt-Bringen eines Stamms nach einem der Ansprüche 8 bis 10 mit der eukaryotischen Zelle.

12. Verfahren zur Herstellung eines chimären Proteins nach Anspruch 5 oder 6 oder eines durch einen Vektor nach einem der Ansprüche 1 bis 4 kodierten chimären Proteins, umfassend die folgenden Schritte:
- einen Stamm nach einem der Ansprüche 8 bis 10 züchten,
- die Sekretion des chimären Proteins induzieren,
- den Kulturüberstand gewinnen.

13. Stamm nach einem der Ansprüche 8 bis 10 für seine Verwendung in der Therapie.


### SEQUENCE LISTING

<110> UNIVERSITE JOSEPH FOURIER
<120> OUTIL DE TRANSFERT ET DE PRODUCTION DE PROTEINES METTANT EN OEUVRE LE SYSTEME DE SECRETION DE TYPE III DE PSEUDOMONAS
<130> U19-B-19682
<150> FR03/13286
   <151> 2003-11-13
<160> 7
<170> PatentIn version 3.1
<210> 1
   <211> 17
   <212> PRT
   <213> Pseudomonas aeruginosa
<400> 1
   Met His Ile Gln Ser Leu Gln Gln Ser Pro Ser Phe Ala Val Glu Leu
   1               5                   10                  15
   His
<210> 2
   <211> 30
   <212> PRT
   <213> Pseudomonas aeruginosa
<400> 2
   Met His Ile Gln Ser Leu Gln Gln Ser Pro Ser Phe Ala Val Glu Leu
   1               5                   10                  15
   His Gln Ala Ala Ser Gly Arg Leu Gly Gln Ile Glu Ala Arg
               20                  25                  30
<210> 3
   <211> 42
   <212> PRT
   <213> Pseudomonas aeruginosa
<400> 3
   Met His Ile Gln Ser Leu Gln Gln Ser Pro Ser Phe Ala Val Glu Leu
   1               5                   10                  15
   His Gln Ala Ala Ser Gly Arg Leu Gly Gln Ile Glu Ala Arg Gln Val
               20                  25                  30
   Ala Thr Pro Ser Glu Ala Gln Gln Leu Ala
           35                  40
<210> 4
   <211> 48
   <212> PRT
   <213> Pseudomonas aeruginosa
<400> 4
   Met His Ile Gln Ser Leu Gln Gln Ser Pro Ser Phe Ala Val Glu Leu
   1               5                   10                  15
   His Gln Ala Ala Ser Gly Arg Leu Gly Gln Ile Glu Ala Arg Gln Val
               20                  25                  30
   Ala Thr Pro Ser Glu Ala Gln Gln Leu Ala Gln Arg Gln Asp Ala Pro
           35                  40                  45
<210> 5
   <211> 54
   <212> PRT
   <213> Pseudomonas aeruginosa
<400> 5
   Met His Ile Gln Ser Leu Gln Gln Ser Pro Ser Phe Ala Val Glu Leu
   1               5                   10                  15
   His Gln Ala Ala Ser Gly Arg Leu Gly Gln Ile Glu Ala Arg Gln Val
               20                  25                  30
   Ala Thr Pro Ser Glu Ala Gln Gln Leu Ala Gln Arg Gln Asp Ala Pro
           35                  40                  45
   Lys Gly Glu Gly Leu Leu
       50
<210> 6
   <211> 96
   <212> PRT
   <213> Pseudomonas aeruginosa
<400> 6
   Met His Ile Gln Ser Leu Gln Gln Ser Pro Ser Phe Ala Val Glu Leu
   1               5                   10                  15
   His Gln Ala Ala Ser Gly Arg Leu Gly Gln Ile Glu Ala Arg Gln Val
               20                  25                  30
   Ala Thr Pro Ser Glu Ala Gln Gln Leu Ala Gln Arg Gln Asp Ala Pro
           35                  40                  45
   Lys Gly Glu Gly Leu Leu Ala Arg Leu Gly Ala Ala Leu Val Arg Pro
       50                  55                  60
   Phe Val Ala Ile Met Asp Trp Leu Gly Lys Leu Leu Gly Ser His Ala
   65                  70                  75                  80
   Arg Thr Gly Pro Gln Pro Ser Gln Asp Ala Gln Pro Ala Val Met Ser
                   85                  90                  95
<210> 7
   <211> 129
   <212> PRT
   <213> Pseudomonas aeruginosa
<400> 7
   Met His Ile Gln Ser Leu Gln Gln Ser Pro Ser Phe Ala Val Glu Leu
   1 5 10 15
   His Gln Ala Ala Ser Gly Arg Leu Gly Gln Ile Glu Ala Arg Gln Val
   20 25 30
   Ala Thr Pro Ser Glu Ala Gln Gln Leu Ala Gln Arg Gln Asp Ala Pro
   35 40 45
   Lys Gly Glu Gly Leu Leu Ala Arg Leu Gly Ala Ala Leu Val Arg Pro
   50 55 60
   Phe Val Ala Ile Met Asp Trp Leu Gly Lys Leu Leu Gly Ser His Ala
   65 70 75 80
   Arg Thr Gly Pro Gln Pro Ser Gln Asp Ala Gln Pro Ala Val Met Ser
   85 90 95
   Ser Ala Val Val Phe Lys Gln Met Val Leu Gln Gln Ala Leu Pro Met
   100 105 110
   Thr Leu Lys Gly Leu Asp Lys Ala Ser Glu Leu Ala Thr Leu Thr Pro
   115 120 125
   Glu

### SEQUENCE LISTING

<110> UNIVERSITE JOSEPH FOURIER
<120> OUTIL DE TRANSFERT ET DE PRODUCTION DE PROTEINES METTANT EN OEUVRE LE SYSTEME DE SECRETION DE TYPE III DE PSEUDOMONAS
<130> U19-B-19682
<150> FR03/13286
   <151> 2003-11-13
<160> 7
<170> PatentIn version 3.1
<210> 1
   <211> 17
   <212> PRT
   <213> Pseudomonas aeruginosa
<400> 1
   Met His Ile Gln Ser Leu Gln Gln Ser Pro Ser Phe Ala Val Glu Leu
   1               5                   10                  15
   His
<210> 2
   <211> 30
   <212> PRT
   <213> Pseudomonas aeruginosa
<400> 2
   Met His Ile Gln Ser Leu Gln Gln Ser Pro Ser Phe Ala Val Glu Leu
   1               5                   10                  15
   His Gln Ala Ala Ser Gly Arg Leu Gly Gln Ile Glu Ala Arg
               20                  25                  30
<210> 3
   <211> 42
   <212> PRT
   <213> Pseudomonas aeruginosa
<400> 3
   Met His Ile Gln Ser Leu Gln Gln Ser Pro Ser Phe Ala Val Glu Leu
   1               5                   10                  15
   His Gln Ala Ala Ser Gly Arg Leu Gly Gln Ile Glu Ala Arg Gln Val
               20                  25                  30
   Ala Thr Pro Ser Glu Ala Gln Gln Leu Ala
           35                  40
<210> 4
   <211> 48
   <212> PRT
   <213> Pseudomonas aeruginosa
<400> 4
   Met His Ile Gln Ser Leu Gln Gln Ser Pro Ser Phe Ala Val Glu Leu
   1               5                   10                  15
   His Gln Ala Ala Ser Gly Arg Leu Gly Gln Ile Glu Ala Arg Gln Val
               20                  25                  30
   Ala Thr Pro Ser Glu Ala Gln Gln Leu Ala Gln Arg Gln Asp Ala Pro
           35                  40                  45
<210> 5
   <211> 54
   <212> PRT
   <213> Pseudomonas aeruginosa
<400> 5
   Met His Ile Gln Ser Leu Gln Gln Ser Pro Ser Phe Ala Val Glu Leu
   1               5                   10                  15
   His Gln Ala Ala Ser Gly Arg Leu Gly Gln Ile Glu Ala Arg Gln Val
               20                  25                  30
   Ala Thr Pro Ser Glu Ala Gln Gln Leu Ala Gln Arg Gln Asp Ala Pro
           35                  40                  45
   Lys Gly Glu Gly Leu Leu
       50
<210> 6
   <211> 96
   <212> PRT
   <213> Pseudomonas aeruginosa
<400> 6
   Met His Ile Gln Ser Leu Gln Gln Ser Pro Ser Phe Ala Val Glu Leu
   1               5                   10                  15
   His Gln Ala Ala Ser Gly Arg Leu Gly Gln Ile Glu Ala Arg Gln Val
               20                  25                  30
   Ala Thr Pro Ser Glu Ala Gln Gln Leu Ala Gln Arg Gln Asp Ala Pro
           35                  40                  45
   Lys Gly Glu Gly Leu Leu Ala Arg Leu Gly Ala Ala Leu Val Arg Pro
       50                  55                  60
   Phe Val Ala Ile Met Asp Trp Leu Gly Lys Leu Leu Gly Ser His Ala
   65                  70                  75                  80
   Arg Thr Gly Pro Gln Pro Ser Gln Asp Ala Gln Pro Ala Val Met Ser
                   85                  90                  95
<210> 7
   <211> 129
   <212> PRT
   <213> Pseudomonas aeruginosa
<400> 7
   Met His Ile Gln Ser Leu Gln Gln Ser Pro Ser Phe Ala Val Glu Leu
   1               5                   10                  15
   His Gln Ala Ala Ser Gly Arg Leu Gly Gln Ile Glu Ala Arg Gln Val
               20                  25                  30
   Ala Thr Pro Ser Glu Ala Gln Gln Leu Ala Gln Arg Gln Asp Ala Pro
           35                  40                  45
   Lys Gly Glu Gly Leu Leu Ala Arg Leu Gly Ala Ala Leu Val Arg Pro
       50                  55                  60
   Phe Val Ala Ile Met Asp Trp Leu Gly Lys Leu Leu Gly Ser His Ala
   65                  70                  75                  80
   Arg Thr Gly Pro Gln Pro Ser Gln Asp Ala Gln Pro Ala Val Met Ser
                   85                  90                  95
   Ser Ala Val Val Phe Lys Gln Met Val Leu Gln Gln Ala Leu Pro Met
               100                 105                 110
   Thr Leu Lys Gly Leu Asp Lys Ala Ser Glu Leu Ala Thr Leu Thr Pro
           115                 120                 125
   Glu